# EUROPÄISCHE PATENTSCHRIFT

(11) **EP 1 430 009 B1**
(45) Veröffentlichungstag und Bekanntmachung des Hinweises auf die Patenterteilung: **21.03.2007**
(21) Anmeldenummer: 02798726.2
(22) Anmeldetag: 16.09.2002
(51) Int. Cl.: C07C 2/34, C10M 107/18, C11D 3/20

(54) **VERFAHREN ZUR TRIMERISIERUNG VON ALPHA-OLEFINEN**
METHOD FOR ALPHA-OLEFIN TRIMERIZATION
PROCEDE DE TRIMERISATION D'ALPHA-OLEFINES

(30) Priorität: 15.09.2001 DE 10145619
(43) Veröffentlichungstag der Anmeldung: 23.06.2004
(73) Patentinhaber: BASF Aktiengesellschaft, 67056 Ludwigshafen (DE)
(72) Erfinder: MIHAN, Shahram, 67061 Ludwigshafen (DE)
(74) Vertreter: Pohl, Michael Friedrich
(86) Internationale Anmeldenummer: PCT/EP2002/010377
(87) Internationale Veröffentlichungsnummer: WO 2003/024902

(56) Entgegenhaltungen:
- EP-A- 0 268 214
- EP-A- 0 366 212
- EP-A- 0 562 258
- WO-A-01/96508
- FR-A- 2 488 259
- US-A- 3 576 898

## Beschreibung

Die vorliegende Erfindung betrifft ein Verfahren zur Trimerisierung von α-Olefinen mit 3 oder mehr Kohlenstoffatomen durch Umsetzung eines α-Olefins oder eines Kohlenwasserstoffgemisches welches α-Olefine enthält bei Temperaturen von 0 bis 150°C und Drücken von 1 bis 200 bar in Gegenwart eines Katalysators.

Des Weiteren betrifft die Erfindung die so erhältlichen Trimeren, die über die Trimeren durch Hydrierung zugänglichen Alkane, die aus den Trimeren erhältlichen Oxoalkohole, die aus diesen Oxoalkoholen erhältlichen Weichmacher und Tenside sowie Schmierstoffe und Kraftstoffadditive, welche die Trimeren und/oder die Alkane enthalten.

Olefintrimere mit bis zu 30 Kohlenstoffatomen haben große wirtschaftliche Bedeutung als Copolymere für Kunststoffe bzw. als Vorprodukte für Oxoalkohole, wobei letztere wiederum Bestandteil von Tensiden und Weichmachern für Kunststoffe sind. Ein weiteres Einsatzgebiet für solche Trimeren sind Schmierstoffe und Kraftstoffadditive. Im Produktverbund der chemischen Industrie sind damit Trimerisierungsverfahren ein zentraler Schritt von großtechnischen Olefinströmen, die etwa den Steamcrackern entstammen, zu Produkten des täglichen Bedarfs.

Aus Makromol. Chem., Rapid Communic. 1992, 13, 277 und Organometallics 1993, 12, 4473 ist bekannt, Ethen und Propen in Gegenwart des Katalysatorsystems aus B(C₆F₅)₃ und Cp'MR₃ (Cp'= C₅H₅ or C₅Me₅, M = Ti oder Zr, R = Me oder CH₂Ph) zu polymerisieren.

Ferner ist aus Organometallics 1996, 15, 693, J. Organomet. Chem. 1997, 548, 23, Macromolecules 1995, 28, 8021 und J. Chem. Soc., Chem. Commun. 1995, 1065 bekannt, das Katalysatorsystem aus η⁵-C₅Me₅TiMe₃ und B(C₆F₅)₃ bei der Polymerisation von Olefinen und Styrol sowie von Isobuten einzusetzen.

J. Organomet. Chem. 592 (1999), 84-94 lehrt die Verwendung von Komlexen vom Typ (η⁵-C₅H₄R)TiCl₃ mit R=CMe₂Ph, CMe₂CH₂Ph, SiMe₂Ph, CHPh₂), (η⁵-C₅H₄CMe₂CH₂Ph)ZrCl₃ · dme und (η⁵-C₅H₄CHPh₂)Ti(CH₂Ph)₃ bei der Polymerisation von Propen.

In Macromolecules 2000, 33, 2807-2814 und Angew. Chem. 2000, 112, Nr. 23, 4519-4521 wird berichtet, dass das System [C₅Me₅TiMe₃]/B(C₆F₅)₃ die Cotrimerisierung von zwei Ethen- mit einem Styrolmolekül katalysiert, wohingegen die Trimerisierung eines α-Olefins als solchem damit nicht gelingt.

Das Katalysatorsystem aus η⁵-C₅Me₅TiMe₃ und B(C₆F₅)₃ findet gemäß Macromolecules 1999, 32, 4491-4493 auch Verwendung bei der gezielten Herstellung butylverzweigter Polyethylene ausgehend von Ethen. Dabei wird 1-Hexen als Nebenprodukt beobachtet.

EP-A-0562258 offenbart ein Verfahren zur Übergangsmetallkomplex-katalysierten Oligomerisierung von Propen.

Aus Angew. Chem. 2001, 113, Nr. 13, 2584-2587 ist bekannt, dass [(η⁵-C₅H₄CMe₃)TiCl₃]/Methylalumoxan ein Polymerisationskatalysator, [(η⁵-C₅H₄CMe₂Phenyl)TiCl₃]/Methylalumoxan dagegen ein Katalysator für die Trimerisierung von Ethen zu 1-Hexen ist.

Die mit den bekannten Verfahren an α-Olefinen mit 3 oder mehr Kohlenstoffatomen erzielten Trimerisierungs-Ergebnisse können jedoch noch nicht befriedigen.

Der vorliegenden Erfindung lag daher ein solches Verfahren zur selektiveren Trimerisierung von α-Olefinen als Aufgabe zugrunde.

Demgemäß wurde ein Verfahren zur Trimerisierung von α-Olefinen mit 3 oder mehr Kohlenstoffatomen durch Umsetzung eines α-Olefins oder eines Kohlenwasserstoffgemisches welches α-Olefine enthält bei Temperaturen von 0 bis 150°C und Drücken von 1 bis 200 bar in Gegenwart eines Katalysators gefunden, bei dem man einen Katalysator verwendet, welcher erhältlich ist aus
a) einer Verbindung RMX₃, in der
   R für eine Cyclopentadienylgruppe steht, deren Wasserstoffatome teilweise oder vollständig durch gleiche oder verschiedene Alkylgruppen und/oder Arylgruppen substituiert sein können, wobei 2 Substituenten gemeinsam auch eine gesättigte oder ungesättigte Kohlenwasserstoffkette bilden können,
   M Titan, Zirkonium oder Hafnium bedeutet,
   die Gruppen X unabhängig voneinander für abstrahierbare Gegenionen stehen, und
b) mindestens einem aktivierenden Zusatzstoff

Des Weiteren wurden die so erhältlichen Trimeren, die über die Trimeren durch Hydrierung zugänglichen Alkane, die aus den Trimeren erhältlichen Oxoalkohole, die aus diesen Oxoalkoholen erhältlichen Weichmacher und Tenside sowie Schmierstoffe und Kraftstoffadditive, welche die Trimeren und/oder die Alkane enthalten, gefunden.

Mit dem erfindungsgemäßen Verfahren lassen sich Trimere von α-Olefinen mit 3 oder mehr Kohlenstoffatomen in hohen Ausbeuten bei hoher Selektivität gewinnen.

Bevorzugte α-Olefine mit 3, 4 oder mehr Kohlenstoffatomen sind geradkettige und verzweigte α-Olefine mit 3 bis 22 und vorzugsweise 4 bis 12 Kohlenstoffatomen. Besonders bevorzugt sind 1-Propen, 1-Penten, 1-Hexen und 1-Decen und ganz besonders bevorzugt 1-Buten.

Es können auch Gemische der α-Olefine untereinander und/oder mit Alkanen eingesetzt werden. Bevorzugt setzt man beim erfindungsgemäßen Verfahren α-Olefin-Ströme ein, welche im wesentlichen ein einziges α-Olefin und daneben ein oder mehrere Alkane enthalten. Besonders bevorzugt ist der Einsatz eines einzigen α-Olefins als solchem.

Als Gruppen R in den Verbindungen RMX₃ sind Cyclopentadienylgruppen bevorzugt, die durch untereinander gleiche oder verschiedene durch untereinander gleiche oder verschiedene C₁- bis C₂₂-Alkylgruppen wie die Methylgruppe substituiert sein können sowie solche Cyclopentadienylgruppen, in denen 2, vorzugsweise benachbarte Kohlenstoffatome des Cyclopentadienylrings durch eine gesättigte oder ungesättigte Kohlenwasserstoffkette mit vorzugsweise 4 C-Atomen, wie in Indenyl und 1,2,3,4-Tetrahydroindenyl miteinander verbunden sind. Besonders bevorzugte Gruppen R sind die Pentamethylcyclopentadienylgruppe, die Isopropylcyclopentadienylgruppe, die Tetramethylcyclopentadienylgruppe und ganz besonders bevorzugt die unsubstituierte Cyclopentadienylgruppe.

Bevorzugte Katalysatoren im erfindungsgemäßen Verfahren sind solche, in denen M für Titan steht.

Bevorzuge Gruppen X in den Verbindungen RMX₃ sind:
- Halogen wie Fluor, Brom, Jod und vor allem Chlor,
- Tosylat, Triflat, Tetrafluoroborat, Tetrakis(pentafluorophenyl)borat, Hexafluorophosphat, Hexafluoroantimonat, Tetraphenylborat,
- C₁- bis C₁₀-Carboxy, vor allem 2-Ethylhexanoat,
- Alkyl-, Arylalkyl- und Arylgruppen wie Methyl, Ethyl, i-Propyl, Phenyl, Benzyl,
- sperrige, nichtkoordinierende Anionen wie B(C₆F₅)₄-.

Die Gruppen X wählt man insbesondere so aus, dass die sie enthaltenden Verbindung RMX₃ eine gute Löslichkeit im gegebenenfalls verwendeten Lösungsmittel haben.

Besonders bevorzugte Gruppen X sind Chlor und Tosylat, insbesondere Chlor.

Neben den Verbindungen RMX₃ kommen auch deren Komplexe mit schwach gebundenen neutralen Komplexliganden in Betracht, z.B. Komplexe vom Typ [RML₃]X₃ mit L beispielsweise in den Bedeutungen Tetrahydrofuran oder Triethylamin und X beispielsweise in den Bedeutungen Tetrafluoroborat, Hexafluoroantimonat oder Tetrakis(pentafluorophenyl)borat.

Hier und im Folgenden weisen Alkyl und Alkoxy in der Regel 1 bis 20, vorzugsweise 1 bis 8 und speziell 1 bis 4 Kohlenstoffatome auf. Beispiele für Alkyl sind insbesondere Methyl, Ethyl, n-Propyl, Isopropyl, n-Butyl, 2-Butyl, Isobutyl, tert.-Butyl, n-Pentyl, n-Hexyl, 2-Hexyl, n-Heptyl, 2-Heptyl, n-Octyl, 2-Octyl und 2-Ethylhex-1-yl. Beispiele für Alkoxy sind insbesondere Methoxy, Ethoxy, n-Propoxy, Isopropoxy, n-Butoxy, 2-Butoxy, Isobutoxy, tert.-Butoxy, n-Pentyloxy, n-Hexyloxy, 2-Hexyloxy, n-Heptyloxy, 2-Heptyloxy, n-Octyloxy, 2-Octyloxy und 2-Ethylhexyl-1-oxy.

Im erfindungsgemäßen Verfahren liegt die Konzentration der Verbindung RMX₃ normalerweise im Bereich von 1 x 10⁻⁷ bis 1, vorzugsweise von 1 x 10⁻⁶ bis 0,1 und insbesondere von 1 x 10⁻⁵ bis 0,01 mol pro kg der Reaktionsmischung.

Die Konzentration an Aktivatorverbindung liegt normalerweise im Bereich von 1 x 10⁻⁸ bis 500, vorzugsweise von 1 x 10⁻⁷ bis 10, insbesondere von 5 x 10⁻⁵ bis 2 und speziell bis 0,5 mol pro kg der Reaktionsmischung, im Falle des Alkylalumoxans gerechnet als mol Al/kg. Die Konzentration an Aktivatorverbindung optimiert der Fachmann im Übrigen vor allem im Hinblick auf Verfahrensführung, verwendeten Reaktor und Reinheit der Einsatzstoffe. Es wurde beobachtet, dass die Menge an Metallalkyl bei größeren Ansätzen vergleichsweise anteilig kleiner ist als bei kleineren Ansätzen.

Als aktivierende Zusatzstoffe eignen sich beispielsweise Metallalkyle deren Alkylgruppen teilweise durch Halogen und/oder Alkoxy ersetzt sein können, weiterhin Alkylalumoxane, Borverbindungen und Mischungen der vorgenannten Aktivatoren, beispielsweise Kombinationen von Metallalkylen, deren Alkylgruppen teilweise durch Halogen und/oder Alkoxy ersetzt sein können, mit Borverbindungen und Kombinationen von Alkylalumoxanen mit Borverbindungen.

Das Molverhältnis von Metall M zu Aktivatorverbindung - im Falle von Alkylalumoxan das Molverhältnis von Aluminium zu Metall M - liegt üblicherweise im Bereich von 1 : 10 bis 1 : 20000, bevorzugt 1 : 50 bis 1 : 1000, insbesondere 1 : 200 bis 1 : 700 und speziell bis 1:500.

Unter den Metallalkylen, die als aktivierende Zusatzstoffe eingesetzt werden, sind die Aluminiumalkyle bevorzugt. Aluminiumalkyle, deren Alkylgruppen teilweise durch Halogen und/oder Alkoxy ersetzt sein können, sind beispielsweise Aluminiumalkyle der Formeln AlR₃, AlR₂Hal, AlRHal₂, AlR₂OR', AlRHalOR' und Al₂R₃Hal₃ und deren Gemische, in denen R und R' unabhängig voneinander für Methyl, Ethyl oder eine geradekettige oder verzweigte C₃- bis C₈-Al kylgruppe stehen und in denen Hal für Fluor, Brom, Iod und vor allem Chlor steht, beispielsweise Trimethylaluminium, Triethylaluminium, Tri-n-propylaluminium, Tri-iso-propylaluminium, Tributylaluminium, Diethylaluminiumchlorid, Diethylaluminiumbromid, Diethylaluminiumethoxid, Diethylaluminiumphenoxid, Ethylaluminiumethoxichlorid. Vorzugsweise werden Aluminiumalkyle vom Typ AlR₃ und AlRHal₂ eingesetzt, wobei Triethylaluminium für sich allein sowie ein Gemisch von Triethylaluminium und Ethylaluminiumdichlorid besonders bevorzugt sind.

Alternativ zu Metallalkylen, deren Alkylgruppen teilweise durch Halogen und/oder Alkoxy ersetzt sein können, können auch Gemische aus den entsprechenden Metallalkylen und geeigneten Cokatalysatoren eingesetzt werden, aus denen sich die Metallalkyle, deren Alkylgruppen teilweise durch Halogen und/oder Alkoxy ersetzt sein können, im Reaktor in situ bilden.

Als Cokatalysatoren eignen sich hierbei Alkylhalogenide, Alkylsiliciumhalogenide und lewissaure Metallhalogenide wie Zinntetrachlorid, Germaniumchlorid, Aluminiumtrichlorid und Titantetrachlorid. Bevorzugte Cokatalysatoren umfassen n-Butylchlorid, n-Butyliodid, Trimethylsilylchlorid, Trimethysilylbromid, Zinntetrachlorid, Germaniumchlorid und vor allem n-Butylbromid.

In dem System aus Metallalkyl, dessen Alkylgruppen teilweise durch Halogen und/oder Alkoxy ersetzt sein können, und Cokatalysator liegen die beiden Komponenten im molaren Verhältnis 1 : 3 bis 30 : 1, vorzugsweise 1 : 1 bis 15 : 1 vor.

Im erfindungsgemäßen Verfahren liegt die Menge der Verbindung RMX₃ normalerweise im Bereich von 1 x 10⁻⁷ bis 1, vorzugsweise von 1 x 10⁻⁶ bis 0,1 und insbesondere von 1 x 10⁻⁵ bis 0,01 mol pro kg der Reaktionsmischung.

Die Menge des Metallalkyls, dessen Alkylgruppen teilweise durch Halogen und/oder Alkoxy ersetzt sein können, liegt normalerweise im Bereich von 1 x 10⁻⁸ bis 500, vorzugsweise von 1 x 10⁻⁷ bis 10 und insbesondere von 5 x 10⁻⁵ bis 0,5 mol pro kg der Reaktionsmischung.

Bevorzugte Trimerisierungskatalysatoren gemäß der Erfindung sind weiterhin solche, die als aktivierenden Zusatzstoff ein Alkylalumoxan enthalten.

Geeignete Alkylalumoxane sind beispielsweise bekannt aus der DE-A 30 07 725, wobei ihre Strukturen weitgehend unaufgeklärt sind. Es handelt sich um Produkte der vorsichtigen partiellen Hydrolyse von Aluminiumalkylen (vgl. DE-A 30 07 725). Diese Produkte liegen offenbar nicht rein vor, sondern als Gemische von offenkettigen und cyclischen Strukturen des Typs IIa und IIb, die miteinander vermutlich im dynamischen Gleichgewicht stehen.

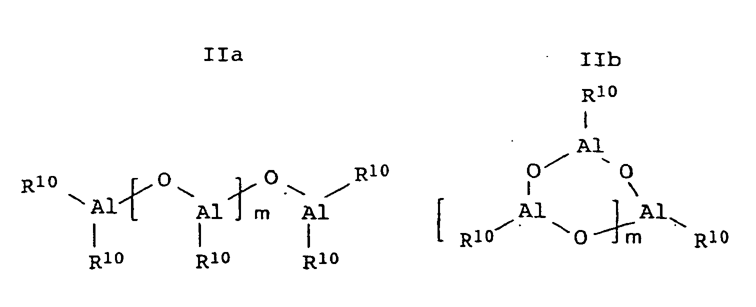

In den Formeln IIa und IIb sind die Gruppen R¹⁰ gleich oder verschieden und stehen unabhängig voneinander für C₁-C₁₂-Alkyl wie Methyl, Ethyl, n-Propyl, i-Propyl, n-Butyl, i-Butyl, sec.-Butyl, tert.-Butyl, n-Pentyl, i-Pentyl, sec.-Pentyl, neo-Pentyl, 1,2-Dimethylpropyl, i-Amyl, n-Hexyl, i-Hexyl, sec.-Hexyl, n-Heptyl, i-Heptyl, n-Octyl, n-Nonyl, n-Decyl, und n-Dodecyl; bevorzugt C₁-C₆-Alkyl wie Methyl, Ethyl, n-Propyl, i-Propyl, n-Butyl, i-Butyl, sec.-Butyl, tert.-Butyl, n-Pentyl, i-Pentyl, sec.-Pentyl, neo-Pentyl, 1,2-Dimethylpropyl, i-Amyl, n-Hexyl, i-Hexyl, sec.-Hexyl, besonders bevorzugt ist Methyl. m ist eine ganze Zahl von 0 bis 40, bevorzugt von 0 bis 25 und besonders bevorzugt von 0 bis 22.

In der Literatur werden auch käfigartige Strukturen für Alumoxane diskutiert (vgl. Organometallics 1996, 15, Seiten 2213-26; Macromol. Symp. 1995, 97, Seiten 15-25).

Ihre Wirksamkeit als aktivierender Zusatzstoff im Sinne der vorliegenden Erfindung erfüllen die Alkylalumoxane unabhängig von ihrer strukturellen Beschaffenheit.

Die Konzentration an Alkylalumoxan liegt normalerweise im Bereich von 1 x 10⁻⁸ bis 500, vorzugsweise von 1 x 10⁻⁷ bis 10 und insbesondere von 5 x 10⁻⁵ bis 2 und speziell bis 0,5 mol pro kg der Reaktionsmischung. Das Verhältnis von Verbindung RMX₃ zum aktivierenden Alkylalumoxan beträgt in der Regel 1 : 10 bis 1 : 20000, bevorzugt 1 : 50 bis 1 : 1000, insbesondere 1 : 200 bis 1 : 700 und speziell bis 1:500, gerechnet als Molverhältnis von Metall M zu Aluminium.

Beispiele für als Aktivatoren geeignete Borverbindungen sind Triarylborane und Salze von Tetrakisarylboraten, vorzugsweise solche mit elektronenziehenden Arylresten. Hierbei steht Aryl vorzugsweise für carboncyclisches Aryl wie insbesondere Phenyl, das vorzugsweise 1, 2, 3, 4 oder 5 elektronenziehende Substituenten wie Fluor und Perfluoralkyl wie Trifluormethyl (bzw. Perfluormethyl) aufweist. Als Gegenionen für die Borate sind insbesondere tertiäre und quartäre Ammoniumionen wie Dibutylammonium und N,N-Dimethylanilinium, sowie das Trityliumion (= [(C₆H₅)₃C]⁺) bevorzugt. Beispiele für bevorzugte elektronenziehende Arylreste sind Pentafluorphenyl und 3,5-Bis(perfluormethyl)phenyl. Beispiele für besonders bevorzugte Borverbindungen mit elektronenziehenden Resten sind Trispentafluorphenylboran, N,N-Dimethylanilinium-tetrakis-pentafluorphenylborat, Tri-n-butylammonium-tetrakis-pentafluorphenylborat, N,N-Dimethylanilinium-tetrakis-(3,5-bisperfluormethyl)-phenylborat, Tri-n-butylammonium-tetrakis-(3,5-bisperfluormethyl)-phenylborat sowie Tritylium-tetrakispentafluorphenylborat). Diese Borverbindungen sind aus EP-A 468 537 sowie EP-A 426 638 bekannt. Bevorzugt sind Tritylium-tetrakispentafluorphenylborat, Trispentafluorphenylboran und vor allem Dimethylanilinium-tetrakis-(pentafluorophenyl)-borat.

In einer weiteren bevorzugten Ausführungsform des erfindungsgemäßen Verfahrens verwendet man einen Trimerisierungskatalysator, der als aktivierenden Zusatzstoff mindestens eine Borverbindung und mindestens ein Aluminiumalkyl, dessen Alkylgruppen teilweise durch Halogen und/oder Alkoxy ersetzt sein können, und/oder mindestens ein Alkylalumoxan enthält.

In diesen Ausführungsformen eignen sich insbesondere die vorgenannten Borverbindungen mit elektronenziehenden Resten (z.B. Trispentafluorphenylboran, N,N-Dimethylanilinium-tetrakis-pentafluorphenylborat, Tri-n-butylammonium-tetrakis-pentafluorphenylborat, N,N-Dimethylanilinium-tetrakis-(3,5-bisperfluormethyl)-phenylborat, Tri-n-butylammonium-tetrakis-(3,5-bisperfluormethyl)-phenylborat sowie Tritylium-tetrakispentafluorphenylborat). Diese Borverbindungen sind aus EP-A 468 537 sowie EP-A 426 638 bekannt. Bevorzugt sind Tritylium-tetrakispentafluorphenylborat, Trispentafluorphenylboran und vor allem Dimethylanilinium-tetrakis-(pentafluorophenyl)-borat.

Die verwendete Menge der aktivierenden Borverbindung ist abhängig von deren Natur. Das Molverhältnis von Verbindung RMX₃ zur aktivierenden Borverbindung beträgt in der Regel 1 : 1 bis 1 : 50, bevorzugt 1 : 1 bis 1 : 10. Bezüglich der Konzentration von RMX₃ und Metallalkyl bzw. Alkylalumoxan sowie bezüglich des Molverhältnisses von RMX₃ zu Metallalkyl bzw. Alkylalumoxan gilt das oben Gesagte.

Als Aluminiumalkyl, dessen Alkylgruppen teilweise durch Halogen und/oder Alkoxy ersetzt sein können, sowie als Alkylalumoxan eignen sich die weiter oben angeführten Vertreter dieser Stoffklassen in den dort angegebenen Mengen bezogen auf die Verbindung RMX₃.

Die Herstellung der Katalysatoren ist im Übrigen allgemein bekannt (vgl. Organometallics 1989, 8, 476 und in Macromolecules 2000, 33, Seite 2813 unter "Experimental Section" zitierte Literatur) und bedarf daher keiner weiteren Erläuterungen. Die Katalysatoren sind auch teilweise kommerziell erhältlich (beispielsweise bei Fa. Aldrich).

Das erfindungsgemäße Verfahren wird in der Regel in einem Lösungsmittel durchgeführt. Vorzugsweise handelt es sich um aprotische Lösungsmittel. Insbesondere weisen die Lösungsmittel keine oder nur äußerst geringe Wasser und/oder Alkoholspuren auf. Beispiele für geeignete Lösungsmittel umfassen geradkettige, verzweigte oder alicyclische gesättigte Kohlenwasserstoffe mit 1 bis 20 Kohlenstoffatomen wie Butan, Pentan, 3-Methylpentan, Hexan, Heptan, 2-Methylhexan, Octan, Cyclohexan, Methylcyclohexan, 2,2,4-Trimethylpentan, Decalin, geradkettige oder verzweigte halogenierte Kohlenwasserstoffe wie Dichlorethan, aromatische Kohlenwasserstoffe wie Benzol, Toluol, Xylol, Ethylbenzol, Mesitylen, Tetralin und die unter den Reaktionsbedingungen flüssigen oligomeren Reaktionsprodukte eingesetzt werden. Geeignet sind auch Gemische der vorgenannten Lösungsmittel. Bevorzugte Lösungsmittel sind aromatische Kohlenwasserstoffe und Mischungen aprotischer Lösungsmittel, die überwiegend, d.h. zu mehr als 50 Vol.-% und insbesondere zu mehr als 90 Vol.-% aus aromatischen Kohlenwasserstoffen und insbesondere zu mehr als 50 Vol.-% und insbesondere zu mehr als 90 Vol.-% aus Toluol bestehen.

Geeignete Lösungsmittel, insbesondere bei der Verwendung eines Trimerisierungskatalysators, umfassend RMX₃ und Alkylalumoxan, sind aprotische Lösungsmittel, z.B. die oben als Lösungsmittel angeführten aliphatischen und insbesondere die aromatischen Kohlenwasserstoffe, und vor allem Toluol und Mischungen aprotischer Lösungsmittel, die zu mehr als 50 Vol.-% und insbesondere zu mehr als 90 Vol.-% aus aromatischen Kohlenwasserstoffen und insbesondere zu mehr als 50 vol.-% und insbesondere zu mehr als 90 Vol.-% Toluol bestehen.

Geeignete Lösungsmittel bei Verwendung eines Trimerisierungskatalysator, der als aktivierenden Zusatzstoff mindestens eine Borverbindung und mindestens ein Aluminiumalkyl, dessen Alkylgruppen teilweise durch Halogen und/oder Alkoxy ersetzt sein können, umfasst, sind aprotische Lösungsmittel, z.B. die weiter oben angeführten als Lösungsmittel angeführten aliphatischen oder aromatischen Kohlenwasserstoffe, und vor allem Toluol und die Mischungen, die zu mehr als 50 Vol.-% und insbesondere zu mehr als 90 Vol.-% aus aromatischen Kohlenwasserstoffen und insbesondere zu mehr als 50 Vol.-% und insbesondere zu mehr als 90 Vol.-% Toluol enthalten.

Insbesondere wegen der Hydrolyseneigung vor allem der Metallalkyle und insbesondere der Aluminiumverbindungen und gegebenenfalls der Cokatalysatoren ist die Trimerisierung in der Regel unter weitestgehendem Feuchtigkeitsausschluss durchzuführen. Dabei kommen an sich bekannte Arbeitstechniken zur Anwendung. Vorzugsweise arbeitet man mit ausgeheizten Apparaturen und unter Schutzgas. Als Schutzgase können alle unter den Reaktionsbedingungen chemisch inerten Gase, zweckmäßigerweise Stickstoff oder Argon verwendet werden. Daneben kann das umzusetzende α-Olefin selbst die Funktion des Schutzgases übernehmen, sofern es unter den Reaktionsbedingungen einen hinreichend hohen Dampfdruck hat.

Die Trimerisierung wird vorzugsweise bei einer Temperatur im Bereich von 1 bis 120 und insbesondere bei 20 bis 110°C durchgeführt. Der Druck liegt vorzugsweise im Bereich von 1 bis 120 bar. Der Druck wird dabei zweckmäßigerweise so gewählt, dass bei der eingestellten Temperatur das Einsatzgemisch flüssig vorliegt. Weiterhin kann der Fachmann mit wenigen routinemäßig durchgeführten Versuchen bei Temperaturen und Drücken in den angegebenen Bereichen und durch Einstellen einer jeweils angepassten Wärmeabfuhr leicht eine optimale Produktivität einstellen.

Bei α-Olefinen, die bei der Reaktionstemperatur gasförmig vorliegen, kann man die Reaktion sowohl drucklos als auch unter erhöhtem Druck betreiben. Bei der drucklosen Durchführung wird man üblicherweise das α-Olefin durch eine Lösung des Katalysator in einem geeigneten Lösungmittel, vorzugweise unter Durchmischen, leiten. Bei der Variante unter Druck wird man vorzugsweise einen Druck anwenden, bei dem das in kondensierter, d.h. flüssiger Phase vorliegt. Es hat sich häugig als vorteilhaft erwiesen, zur Einstellung des Drucks zusätzlich noch ein Inertgas wie Stickstoff zu verwenden.

Das erfindungsgemäße Verfahren kann diskontinuierlich oder kontinuierlich durchgeführt werden, wobei im technischen Maßstab der kontinuierlichen Fahrweise der Vorzug gegeben wird.

Für das erfindungsgemäße Verfahren geeignete Reaktoren für die kontinuierliche Reaktionsführung sind dem Fachmann geläufig, etwa aus Ullmann's Enzyklopädie der technischen Chemie, Band 1, 3. Auflage, 1951, Seite 743 ff.; druckfeste Reaktoren sind dort auf Seite 769 ff. beschrieben.

Die übrigen Randbedingungen des Trimerisierungsverfahrens stellt, der Fachmann anhand seines allgemeinen Fachwissens ein (vgl.dazu etwa die DE-A 196 07 888).

Für die Katalysatordesaktivierung bei Umsetzungsende eignen sich beispielsweise Wasser und Monoalkohole mit 1 bis 10 Kohlenstoffatomen, wobei diesen Substanzen Mineralsäuren zugesetzt werden können sowie polare Stoffe, welche gängige Inhibitoren für Übergangsmetall-Katalysatoren sind wie Kohlenmonoxid, Kohlendioxid, Kohlenstoffdisulfid, Schwefelwasserstoff und Ammoniak.

Die Produkte des erfindungsgemäßen Verfahrens werden zweckmäßigerweise destillativ gereinigt.

Um einen hohen Gesamtumsatz zu erreichen, kann unumgesetztes α-Olefin zurückgewonnen und in die Umsetzung zurückgeführt werden.

Die mit dem erfindungsgemäßen Verfahren erhältlichen Trimeren eignen sich in besonderem Maße zur Herstellung von Monoalkoholen für Weichmacher und Tenside. Dazu unterwirft man die Trimeren zweckmäßigerweise der Hydroformylierung, bei der Gemische der um ein Kohlenstoffatom kettenverlängerten Aldehyde und Alkohole entstehen, welche anschließend zu den gewünschten Alkoholen hydriert werden. Die Durchführung der Hydroformylierung und Hydrierung sind dem Fachmann an sich bekannt und bedürfen daher keiner weiteren Erläuterungen.(vgl. z.B. Beller et al., Journal of Molecular Catalysis A 104 (1995) Seiten 17-85).

Ferner können die Trimeren nach an sich bekannten Verfahren zu den entsprechenden Alkanen hydriert werden.

Die folgenden Beispiele erläutern die Erfindung.

### Beispiele 1 bis 9

Cyclopentadienyltitantrichlorid ("CpTiCl₃") wurde in über Natriummetall getrocknetem Toluol bei 40°C gelöst. Zu der so erhaltenen Lösung wurde Methylalumoxan ("MAO", von Firma Witco) in Form einer 4,75-molaren Lösung in Toluol zugesetzt (bezüglich der Mitverwendung von Borat vergleiche die Fußnote 2 zu Tabelle 1, s.u.). In die entstandene Lösung wurde über die Zeitdauer von 60 min bei der Temperatur T ein Überschuss des jeweiligen α-Olefins zudosiert. Danach wurde eine Mischung aus 15 ml konzentrierter Salzsäure und 50 ml Methanol zugegeben und 15 min gerührt. Danach wurden weitere 250 ml Methanol zugesetzt und die Mischung gerührt. Anschließend wurde vom ausgefallenen Polymer filtriert. Das Filtrat wurde mit Wasser gewaschen und über Natriumsulfat getrocknet. Die Zusammensetzung des Filtrats wurde mittels quantitativer Gaschromatographie ermittelt.

Die folgende Tabelle 1 gibt die verwendeten Stoffe, die zugehörigen Stoffmengen sowie die wesentlichen Reaktionsparameter und Versuchsergebnisse wieder.

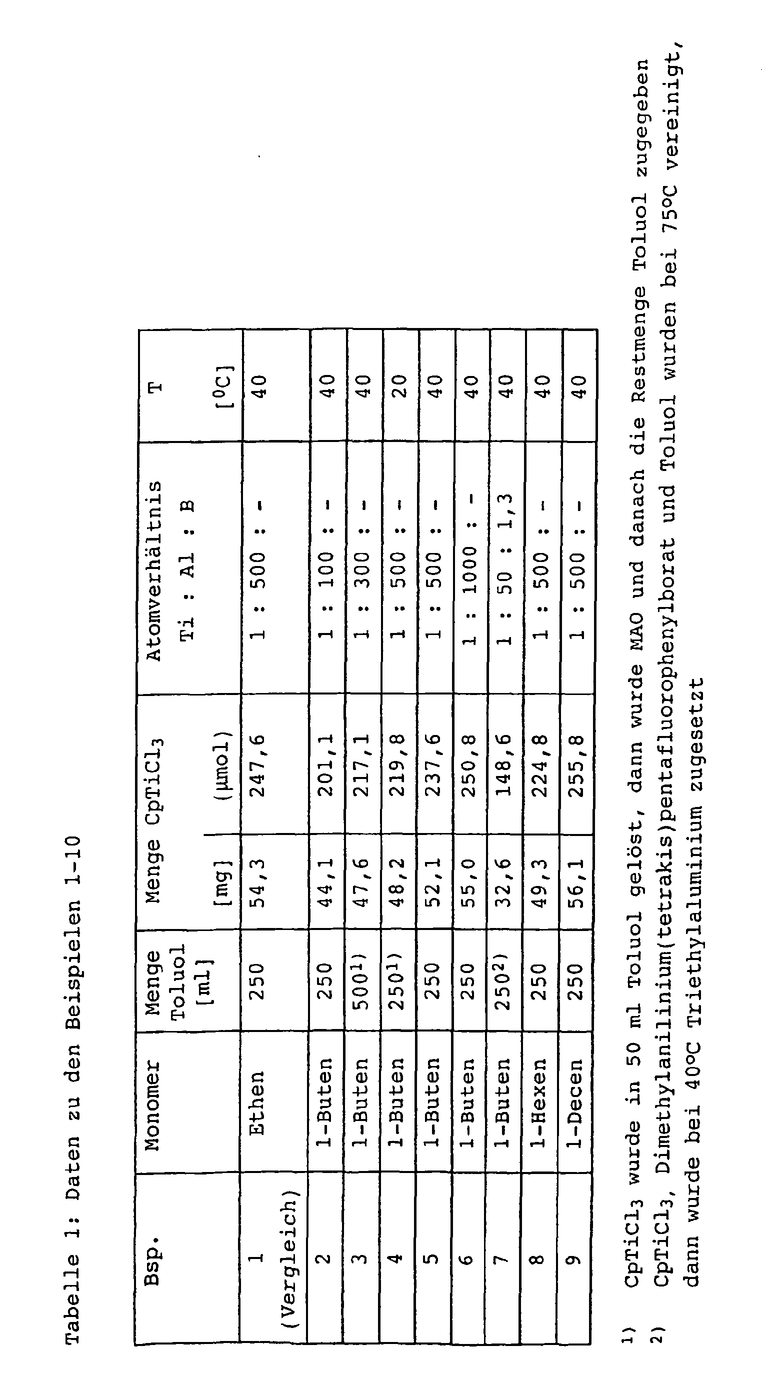

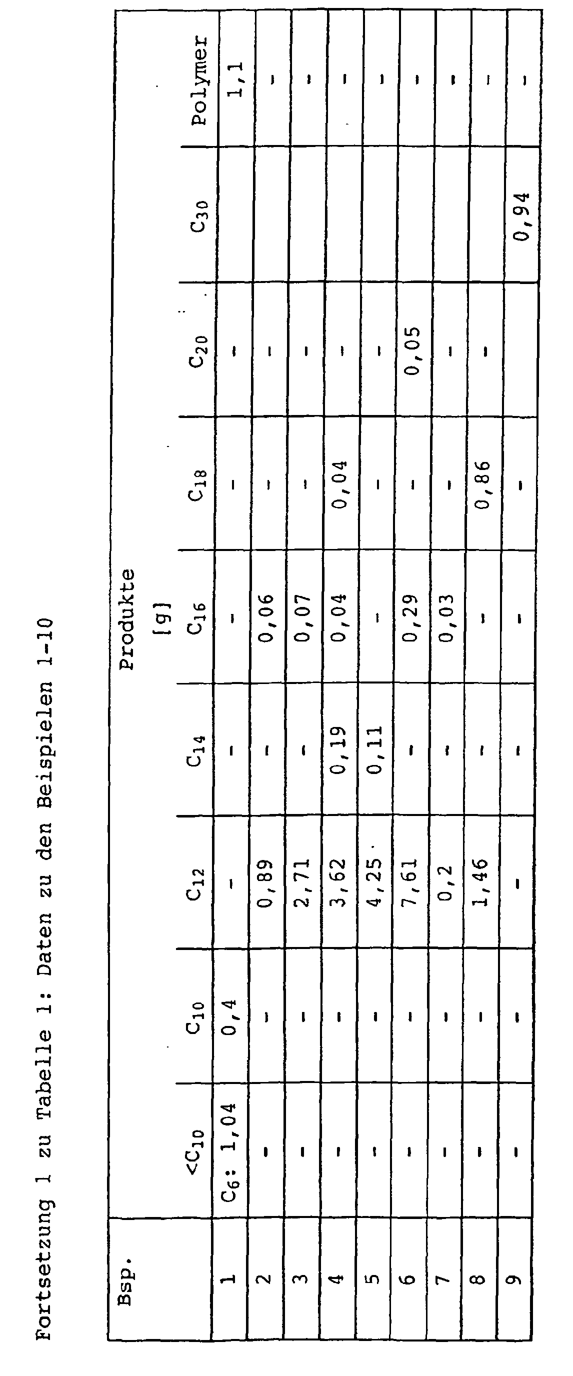

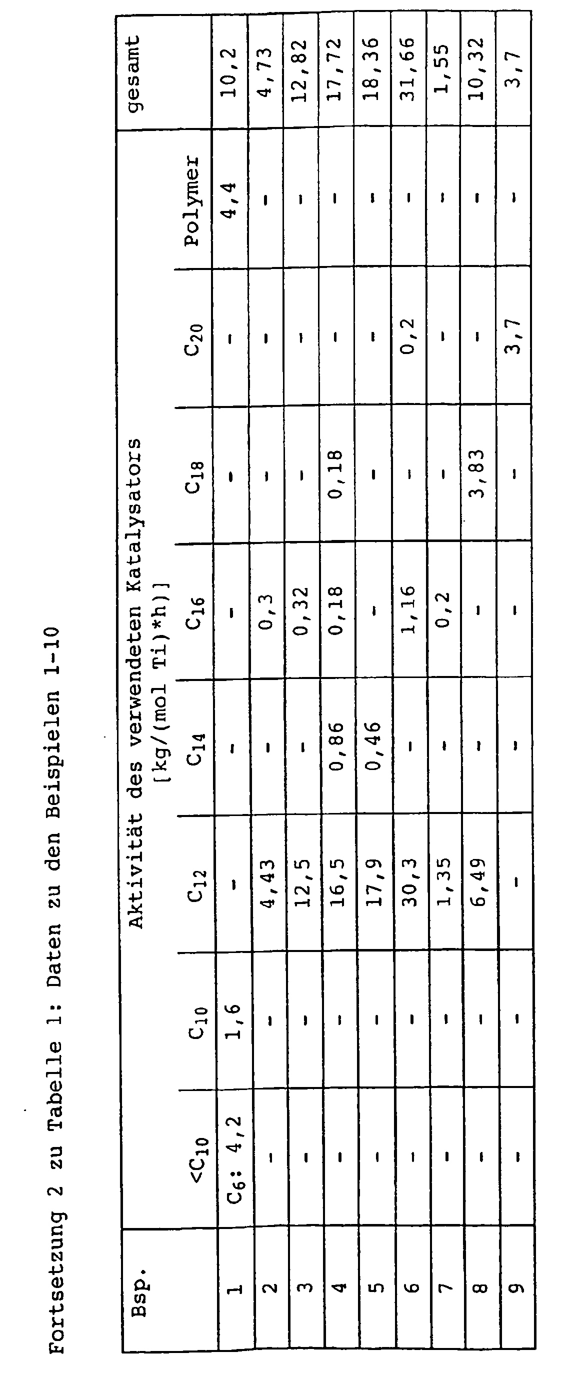

### Beispiel 10

Ein mit Argon gespülter Autoklav wurde im Vakuum 60 min bei 140°C ausgeheizt. Hierin legte man unter Argon-Atmosphäre eine Lösung von 11,4 mg CpTiCl₃ in 5 ml wasserfreiem Toluol (erhältlich von Aldrich) vor und gab dann 6,4 g einer 30 Gew.-% Lösung von Methylalumoxan in Toluol zu. Anschließend gab man in den Autoklaven über eine Schleuse 10 g 1-Buten und stellte mit Stickstoff einen Druck von 10 bar ein und erwärmte auf 40°C. Nach einer Stunde kühlte man ab, entspannte den Autoklaven auf Normaldruck und gab 1,14 g Octan zur Reaktionsmischung. Unter Kühlung mit einem Eisbad gab man zur Desaktivierung 5 ml einer 5 gew.-% wässrigen Salzsäure zu. Die organische Phase wurde in üblicher Weise gaschromatographisch untersucht. Es wurden Dodecene (Isomere) in einer Selektivität von 86 % mit einer Produktivität von 0.75 kg C12-Isomere pro g Titan gebildet.

### Beispiel 11

Analog Beispiel 10 wurde eine Oligomerisierung von 1-Buten durchgeführt wobei man anstelle von CpTiCl₃ 14.4 mg Pentamethylcyclopentadienyltitantrichlorid einsetzte. Es wurden Dodecene (Isomere) in einer Selektivität von 81 % mit einer Produktivität von 0.26 kg C12-Isomere pro g Titan gebildet.

### Beispiel 12

Analog Beispiel 10 wurde eine Oligomerisierung von 1-Buten durchgeführt wobei man anstelle von CpTiCl₃ 6,6 mg Isopropylcyclopentadienyltitantrichlorid einsetzte. Abweichend von Beispiel 10 wurden 1.93 g 30 gew-%ige Methylalumoxan-Lösung und 20 ml 1-Buten eingesetzt und die Reaktion bei 60°C durchgeführt. Es wurden Dodecene (Isomere) in einer Selektivität von 78 % mit einer Produktivität von 0.76 kg C12-Isomere pro g Titan gebildet.

## Patentansprüche

1. Verfahren zur Trimerisierung von α-Olefinen mit 3 oder mehr Kohlenstoffatomen durch Umsetzung eines α-Olefins oder eines Kohlenwasserstoffgemisches welches α-Olefine enthält bei Temperaturen von 0 bis 150°C und Drücken von 1 bis 200 bar in Gegenwart eines Katalysators, **dadurch gekennzeichnet, dass** man einen Katalysator verwendet, welcher erhältlich ist aus
a) einer Verbindung RMX₃, in der
R für eine Cyclopentadienylgruppe steht, deren Wasserstoffatome teilweise oder vollständig durch gleiche oder verschiedene Alkylgruppen und/oder Arylgruppen substituiert sein können, wobei 2 Substituenten gemeinsam auch eine gesättigte oder ungesättigte Kohlenwasserstoffkette bilden können,
M Titan, Zirkonium oder Hafnium bedeutet,
die Gruppen X unabhängig voneinander für abstrahierbare Gegenionen stehen, und
b) mindestens einem aktivierenden Zusatzstoff, der unter Metallalkylen, deren Alkylgruppen teilweise durch Halogen und/oder Alkoxy ersetzt sein können, Alkylalumoxanen, Borverbindungen und Mischungen davon ausgewählt ist.

2. Verfahren nach Anspruch 1, **dadurch gekennzeichnet, dass** man als aktivierenden Zusatzstoff mindestens ein Metallalkyl verwendet, dessen Alkylgruppen teilweise durch Halogen und/oder Alkoxy ersetzt sein können.

3. Verfahren nach Anspruch 2, **dadurch gekennzeichnet, dass** man als aktivierenden Zusatzstoff ein Aluminiumalkyl verwendet, dessen Alkylgruppen teilweise durch Halogen und/oder Alkoxy ersetzt sein können.

4. Verfahren nach Anspruch 1, **dadurch gekennzeichnet, dass** man als aktivierenden Zusatzstoff mindestens eine Borverbindung in Kombination mit mindestens ein Metallalkyl verwendet, dessen Alkylgruppen teilweise durch Halogen und/oder Alkoxy ersetzt sein können.

5. Verfahren nach Anspruch 1, **dadurch gekennzeichnet, dass** der aktivierende Zusatzstoff mindestens ein Alkylalumoxan umfasst.

6. Verfahren nach Anspruch 5, **dadurch gekennzeichnet, dass** der aktivierende Zusatzstoff mindestens eine Borverbindung und mindestens ein Alkylalumoxan umfasst.

7. Verfahren nach den Ansprüchen 1 bis 6, **dadurch gekennzeichnet, dass** man ein α-olefin einsetzt, welches praktisch frei ist von sonstigen Olefinen.

8. Verfahren nach einem der vorhergehenden Ansprüche, **dadurch gekennzeichnet, dass** X für Chlor steht.

## Claims

1. A process for the trimerization of α-olefins of 3 or more carbon atoms by reacting an α-olefin or a hydrocarbon mixture which contains α-olefins at from 0 to 150°C and from 1 to 200 bar in the presence of a catalyst, wherein a catalyst which is obtainable from
a) a compound RMX₃, in which
R is a cyclopentadienyl group, some or all of whose hydrogen atoms can be substituted by identical or different alkyl groups, arylalkyl groups and/or aryl groups, where 2 substituents together can also form a saturated or unsaturated hydrocarbon chain,
M is titanium, zirconium or hafnium and
the groups X, independently of one another, are abstractable counterions, and
b) at least one activating additive, which is selected from among metal alkyls, some of whose alkyl groups may be replaced by halogen and/or alkoxy, alkylaluminoxanes, boron compounds and mixtures thereof
is used.

2. A process as claimed in claim 1, wherein the activating additive used comprises at least one metal alkyl, some of whose alkyl groups may be replaced by halogen and/or alkoxy.

3. A process as claimed in claim 2, wherein the activating additive used is an alkylaluminum, some of whose alkyl groups may be replaced by halogen and/or alkoxy.

4. A process as claimed in claim 1, wherein the activating additive used comprises at least one boron compound in combination with at least one metal alkyl, some of whose alkyl groups may be replaced by halogen and/or alkoxy.

5. A process as claimed in claim 1, wherein the activating additive comprises at least one alkylaluminoxane.

6. A process as claimed in claim 5, wherein the activating additive comprises at least one boron compound and at least one alkylaluminoxane.

7. A process as claimed in any of claims 1 to 6, wherein an α-olefin which is virtually free of other olefins is used.

8. A process as claimed in any of the preceding claims, wherein X is chlorine.

## Revendications

1. Procédé de trimérisation d'α-oléfines comportant 3 atomes de carbone ou davantage par réaction d'une α-oléfine ou d'un mélange d'hydrocarbures qui contient des α-oléfines à des températures de 0 à 150°C et à des pressions de 1 à 200 bars, en présence d'un catalyseur, **caractérisé en ce qu'**on utilise un catalyseur qui peut être obtenu à partir
a) d'un composé RMX₃, où
R représente un groupe cyclopentadiényle, dont les atomes d'hydrogène peuvent être partiellement ou totalement substitués par des groupes alkyle et/ou des groupes aryle identiques ou différents, 2 substituants pouvant aussi former conjointement une chaîne hydrocarbonée saturée ou insaturée,
M représente du titane, du zirconium ou de l'hafnium,
les groupes X représentent indépendemment l'un de l'autre des contre-ions que l'on peut abstraire, et
b) d'au moins un additif activant qui est choisi parmi des métal-alkyles dont les groupes alkyle peuvent être remplacés partiellement par de l'halogène et/ou de l'alcoxy, des alkylalumoxanes, des composés de bore et leurs mélanges.

2. Procédé suivant la revendication 1, **caractérisé en ce que**, comme additif activant, on utilise au moins un métal-alkyle dont les groupes alkyle peuvent être remplacés partiellement par de l'halogène et/ou de l'alcoxy.

3. Procédé suivant la revendication 2, **caractérisé en ce que**, comme additif activant, on utilise un aluminium-alkyle, dont les groupes alkyle peuvent être partiellement remplacés par de l'halogène et/ou de l'alcoxy.

4. Procédé suivant la revendication 1, **caractérisé en ce que**, comme additif activant, on utilise au moins un composé de bore en combinaison avec au moins un métal-alkyle dont les groupes alkyle peuvent être remplacés partiellement par de l'halogène et/ou de l'alcoxy.

5. Procédé suivant la revendication 1, **caractérisé en ce que** l'additif activant comprend au moins un alkylalumoxane.

6. Procédé suivant la revendication 5, **caractérisé en ce que** l'additif activant comprend au moins un composé de bore et au moins un alkylalumoxane.

7. Procédé suivant les revendications 1 à 6, **caractérisé en ce qu'**on met en oeuvre une α-oléfine qui est pratiquement exempte d'autres oléfines.

8. Procédé suivant l'une des revendications précédentes, **caractérisé en ce que** X représente du chlore.
